(19) Europäisches Patentamt
European Patent Office
Office européen des brevets

(11) **EP 1 138 385 B1**

(12) **EUROPEAN PATENT SPECIFICATION**

(45) Date of publication and mention
of the grant of the patent:
**22.08.2007 Bulletin 2007/34**

(21) Application number: **00960981.9**

(22) Date of filing: **13.09.2000**

(51) Int Cl.:
**B01J 23/88** *(2006.01)* **C07C 51/25** *(2006.01)*

(86) International application number:
**PCT/JP2000/006260**

(87) International publication number:
**WO 2001/021307 (29.03.2001 Gazette 2001/13)**

(54) **PREPARATION OF CATALYST AND USE FOR ACRYLIC ACID PRODUCTION**

KATALYSATORHERSTELLUNG UND VERWENDUNG FÜR DIE ACRYLSÄUREHERSTELLUNG

PREPARATION D'UN CATALYSEUR ET UTILISATION POUR LA PRODUCTION D'ACIDE ACRYLIQUIE

(84) Designated Contracting States:
**BE DE FR GB**

(30) Priority: **17.09.1999 JP 26282299**

(43) Date of publication of application:
**04.10.2001 Bulletin 2001/40**

(73) Proprietor: **NIPPON KAYAKU KABUSHIKI KAISHA
Tokyo 102-8172 (JP)**

(72) Inventors:
• **OHISHI, Junzo
Asa-gun, Yamaguchi 757-0002 (JP)**
• **SENYO, Masahiro
Asa-gun, Yamaguchi 757-0002 (JP)**
• **SEO, Yoshimasa
Asa-gun, Yamaguchi 757-0002 (JP)**

• **SUGI, Hideki
Sawa-gun, Gumma 370-1135 (JP)**

(74) Representative: **Gille Hrabal Struck Neidlein Prop Roos
Patentanwälte
Brucknerstrasse 20
40593 Düsseldorf (DE)**

(56) References cited:
**EP-A- 0 492 813      EP-A1- 0 711 745
EP-A1- 0 758 562      FR-A- 2 754 817
JP-A- 9 316 023       JP-A- 11 114 418
JP-A- 11 343 261      US-A- 4 051 180**

Remarks:
The file contains technical information submitted after the application was filed and not included in this specification

**Description**

TECHNICAL FIELD

The present invention relates to process for producing a catalyst, and more particularly for producing a catalyst suitable for use in the production of acrylic acid by vapor-phase catalytic oxidation of acrolein with molecular oxygen.

BACKGROUND ART

As a catalyst for the production of an unsaturated acid by vapor-phase catalytic oxidation of the unsaturated aldehyde, there are known, for examples, the catalyst obtained by tableting a catalytically active component composition, the catalyst obtained by molding a catalytically active component with auxiliary components into a globule or a ring, and the catalyst obtained by supporting a catalytically active component on inactive carriers with binders (hereinafter called coated catalyst).

In FR -A- 2 754 817 catalysts are described which are prepared by drying an aqueous solution of different metal salts. The catalysts are calcined and the calcined oxide product is usually prepared as a powder. It is possible to provide a further component containing an element selected from Na, K, Rb, Cs, P and S on the powdered catalyst. What is generated through this process is a component carried on the metal oxide catalyst. It is mentioned that this catalyst composition may be carried on a further carrier such as silica or alumina.

In an example, a Mo/Sb/V/Nb catalyst is prepared using antimony acetate.

The preparation of catalysts on inert carriers is described in EP -A- 0 711 745. The document refers to the addition of a binder for powder, such as ammonium nitrate, cellulose, starch, polyvinyl alcohol and stearic acid.

For the preparation of the coated catalyst, Japanese Patent Laid-Open No. 11709/1976 (JP 51 011 709) disclosed the method of coating by rolling the active component and the carriers in a rotating drum or jar; Japanese Patent Laid-Open No.153889/1977 (JP 52 153 889) disclosed the method of coating either by spraying the aqueous suspension of a preliminarily calcined active component over the carriers or by spreading the active component over vigorously moving carriers; and Japanese Patent Laid-Open No.85139/1989 (JP 1 085 139) disclosed the method of production using various granulators.

In the industrial plant for producing acrylic acid by vapor-phase catalytic oxidation of acrolein with molecular oxygen, the catalyst is packed into an as long as 5m reaction tube from the upper side. Therefore, if a coated catalyst having a weak mechanical strength is packed, the catalytically active components composition is peeled out and pulverized into powder and this causes the problem that the pressure inside the tube is abnormally raised during the reaction. So, the catalyst is demanded to have a large mechanical strength in property (such as little attrition resistance).

In recent years, the production of acrylic acid by vapor-phase catalytic oxidation of acrolein trends towards "high load reaction condition", that is, to increase the amount of acrolein supplied per unit volume of the catalyst. Since the oxidation reaction of acrolein is exothermic, such increased amount of acrolein brings about hot spots, which are likely to drive out the catalytic components including molybdenum, a constitutional element of the catalyst. As the defect, difference in pressure inside the reaction tube increases with the elapse of reaction time, decreasing the reaction merits (such as conversion of acrolein and acrylic acid yield) and blocking a long operation.

Such a situation demands to develop a highly active catalyst that would enable the operation at low reaction temperature. The present applicant made a diligent study to solve these problems, disclosing in Japanese Patent Laid-Open No.299797/1996 (JP 8 299 797) and EP 0 758 562 filed previous to the present application that the catalyst having a certain X-ray diffraction pattern was highly active and had a large mechanical strength. The description mentions antimony trioxide, antimony pentoxide, antimony acetate and antimony trichloride as examples of antimony compounds which may be used. It also disclosed that it was preferable to use antimony trioxide subjected to no chemical treatment for an antimony-containing compound. <

DISCLOSURE OF THE INVENTION

The present applicant has made another diligent study to solve these problems, finding to complete the present invention that the catalyst obtained by using a certain material is highly active for the oxidation reaction of acrolein and has a large mechanical strength. Namely, the present inventors have studied to reveal that antimony acetate used for the source material can provide the catalyst having larger mechanical strength, high activity and good reproducibility. The present invention relates to an antimony-containing molybdenum catalyst produced by using antimony acetate for an antimony source material, and more particularly to the following:

(1) A process for producing a coated catalyst by coating the catalytically active component which has a composition represented by the formula (1):

$$Mo_{12}V_aW_bCu_cSb_dX_eY_fZ_gO_h \qquad (1)$$

wherein Mo, V, W , Cu, Sb and O represent molybdenum, vanadium, tungsten, copper, antimony and oxygen respectively;

X represents at least one element selected from the group consisting of alkali metals and thallium;

Y represents at least one element selected from the group consisting of magnesium, calcium, strontium, barium and zinc;

Z represents at least one element selected from the group consisting of niobium, cerium, tin, chromium, manganese, iron, cobalt, samarium, germanium, titanium and arsenic; a, b, c, d, e, f, g and h represent atomic ratios of their respective elements, with $0 < a \leq 10$ , $0 \leq b \leq 10$, $0 < c \leq 6$, $0 < d \leq 10$, $0 \leq e \leq 0.5$, $0 \leq f \leq 1$, $0 \leq g < 6$, based on 12 of molybdenum atoms; and h is the number of oxygen atoms required to satisfy the total valence of the other elements, on an inactive carrier,

the process comprising the following steps (a) to (c):

(a) drying either an aqueous solution or a dispersion containing the compounds of the catalytic component elements, wherein the aqueous solution or dispersion contains antimony acetate as the antimony material, thus obtaining a catalytic component composition;

(b) calcining the catalytic component composition obtained in step (a), to prepare a calcined powder; and

(c) coating the calcined powder obtained in step (b) on the carrier, together with crystalline cellulose as a binder and optionally a strength-improver.

(2) The process according to the above (1), wherein the antimony acetate as the antimony source material for the preparation of the aqueous solution or the dispersion has a purity of 99 % or more.

(3) The process according to the above (1) or (2), wherein the supported-calcined powder has 15 to 50% by mass rate based on the total amount of said carrier and said calcined powder in the step (c).

(4) The process according to any of the above (1) to (3), wherein said drying in step (a) is spray-drying.

(5) The process according to any of the above (1) to (4), wherein said strength-improver in the step (c) is ceramic fiber.

(6) Use of the coated catalyst according to any of the above (1) to (5) in a process for the production of acrylic acid by vapor-phase catalytic oxidation of acrolein with molecular oxygen.

The present invention will be described in detail below, where "part" and "%" mean "part by mass" and "% by mass" respectively unless otherwise defined.

There is no limitation to antimony acetate used for the source material in the present invention. Antimony acetate available on the market is satisfactory to use. The purity etc. unless it damages catalytic activity, has no problem to be solved. Antimony acetate having a purity of 95% and more is generally used. One having a purity of 99% and more is more preferable.

The atomic ratios of the respective elements in the catalytically active component produced according to the present invention, if they are within the ranges as described in the above formula (1), have no problem to be solved. The more preferable ranges in the formula (1) are $2 \leq a \leq 5$, $0.2 \leq b \leq 2$, $0.2 \leq c \leq 54$, $0.3 \leq d \leq 4$, $0 \leq e \leq 0.2$, $0 \leq f \leq 0.5$, $0 \leq g \leq 3$.h, the atomic ratio of oxygen element, varies depending on atomic valences and atomic ratios of the other elements and is a value determined exclusively by the calcinations. The range is generally $42 \leq h \leq 133$, preferably $43 \leq h \leq 75$.

The catalyst is produced according to the method of the invention.

For example, at first, an aqueous solution containing the elements (hereinafter called catalytic components) in a catalytically active component composition as shown in formula (1) or an aqueous dispersion of compounds (catalytic component compounds) containing said elements is prepared. The aqueous solution or dispersion is hereinafter referred simply as "a slurry solution" unless otherwise specified. The slurry solution can be generally obtained by mixing the catalytic component compounds homogeneously in water to dissolve or disperse. The slurry solution in the present invention, if it is an aqueous solution, is most preferable. The catalytic component compounds are satisfactorily mixed in a formulation rate that the atomic ratios of the catalytic components fall within their respective ranges in the above formula (1).

The slurry solution can be produced by any procedure, for example, by making up catalytic component compounds into their respective solutions or dispersions, followed by mixing them; by dissolving or dispersing one or more catalytic component compounds to get plural solutions, followed by mixing them; or by making up all the catalytic component compounds into a solution or dispersion by one step.

The slurry solution is generally produced by preparing the A solution containing a molybdenum compound, a vanadium compound, a tungsten compound and antimony acetate and the B solution containing a copper compound, followed by mixing them. X component, Y component and Z component, if contained, are satisfactorily dissolved or dispersed in any adequate solution. Generally, X component and Y component are dissolved or dispersed in the B solution, and Z component is dissolved or dispersed in the A solution or the B solution suitably.

The amount of water used is not particularly limited as long as it is enough to produce the slurry solution, and suitably determined in view of method and temperature to select in a succeeding drying step. It is usually 200 to 2,000 parts by mass based on 100 parts of the total mass of compounds. Too little water cannot completely dissolve (or homogeneously mix) compounds. Too much water may bring about the problem of high energy-cost for drying step or incomplete drying.

The other catalytic component compounds used for producing the catalyst than antimony compound, provided that they can be converted into their respective oxides upon calcination, are not limited to the specific ones in the present invention. They include chlorides, sulfates, nitrates, ammonium salts and oxides of the other catalytic components than antimony. The illustrative examples include: molybdenum trioxide, molybdic acid and the salts thereof as a molybdenum compound; vanadium pentaoxide, vanadyl sulfate, vanadic acid and the salt thereof as a vanadium compound; tungstic acid and the salt thereof as a tungsten compound; and copper oxide, copper sulfate, copper nitrate and copper molybdate as a copper compound. These compounds may be used alone or as a mixture of two or more.

Then, the slurry solution is dried to give the dried powder (catalytic component composition) (step (a)).

The drying method, if it can dry completely the slurry solution, is not particularly limited, and includes drum drying, freeze-drying and spray drying. The spray drying is the most preferable in the present invention because it can dry to change the slurry state into the powder state in a short time. The drying temperature varies depending on the slurry solution concentration and the solution-feeding rate etc., and is about 85 to 130°C at the outlet of the dryer. The drying is preferably carried out in such a way that it may give the dried powder having an average particle size of 20 to 100 $\mu$m.

The dried powder (catalytic component composition) as described above is calcined to give a catalytically active component (step (b)).

The calcination may be carried out before or after a shaping step. The dried powder may be calcined only before the shaping step as the case may be, but is preferably calcined by two steps, i.e. "preliminary calcination" before the shaping step and " primary calcination" after the shaping step as described below. The calcinations can be carried out by the known methods with no limitation.

When the dried powder is calcined by two steps, the preliminary calcination is generally carried out at a temperature of 250 to 500°C, preferably 300 to 450°C, for 1 to 15 hrs, preferably 3 to 6 hrs. The preliminary calcination step has an effect to provide a low attritional catalyst, which prevents the catalytically active component from pulverizing and peeling when a completed catalyst is packed in a reaction tube. The calcination product obtained by the preliminary calcination of the dried powder, including the further pulverized product, is hereinafter called a preliminary calcination powder in the present invention.

When the dried powder is calcined by two steps, the primary calcination is generally carried out at a temperature of 250 to 500°C, preferably 300 to 450°C, for 1 to 50 hrs.

The catalysts of the present invention can be obtained either by shaping the above dried powder (catalytic component composition), followed by calcining; or by calcining the above dried powder to get the calcination powder (usually in a granule), which is then optionally pulverized, followed by shaping by a known adequate way, with further succeeding primary calcining if necessary. A shaping way generally includes (I) compressing into a tablet, (II)mixing with shaping auxiliaries such as silica gel, diatomaceous earth and alumina powder, followed by extruding into a sphere or ring, and (III) coating and supporting on a carrier, preferably on a spherical carrier. The way (III) is the method chosen in the present invention, where it is desirable to coat the above calcination powder on the carrier together with binders and strength-improvers if necessary to get the coated catalyst [step(c)].

The coated catalyst will be described in detail below.

Tumbling granulation mentioned hereinafter is preferable for the coating step. This way is , for example, as follows. The flat or uneven disk mounted to the bottom of a fixed vessel rolls at a high speed, causing the carrier inside the vessel to take an axial rotation and an orbital revolution repeatedly so that it may be stirred vigorously. The mixture of a binder, the preliminary calcination powder and, if necessary, a shaping auxiliary and a strength-improver is added to the stirred carrier to coat the mixture on the carrier.

The binder may be added into the vessel: (1) as the previously blended mixture mentioned above, (2) when a mixture of the other ingredients is added, (3) after the mixture of the other ingredients is added, (4) before the mixture of the other ingredients is added, or (5) by dividing the mixture and the binder into their respective portions, followed by adding them in an adequately combined manner of (2) to (4) to make up the total mass. The manner (5) is preferably carried out by using an automatic feeder to control the addition rate so that a prescribed amount of mixture is supported on the carrier without the mixture being adhered to the vessel wall and being agglomerated by itself.

An illustration of the carrier includes spherical carriers made of, for example, silicone carbide, alumina, mullite or alundum that has a diameter of 2.5 to 10 mm, preferably 3 to 6 mm. Among these carriers, those having a porosity rate of 30 to 50% and a water-absorption rate of 10 to 30% are preferably used.

The ratio of the preliminary calcination powder used for coating on the carrier (supported-calcination powder) is generally 10 to 75%, preferably 15 to 50% relative to the total amount of the preliminary calcination powder and the carrier( supported-calcination powder + carrier).

The coated catalyst, if it contains the powder for coating in a large ratio, has a higher reaction activity, but is likely to have less mechanical strength (a large attrition rate). On the contrary, if it contains the powder for coating in a small ratio, it has more mechanical strength (a less attrition rate), but is likely to have a lower reaction activity.

A binder is used in the present invention when the preliminary calcination powder (the granule obtained by the preliminary calcinations or the pulverized product thereof) is coated on the carrier. Crystalline cellulose in the celluloses is the binder which is chosen The cellulose makes to give a good shaping, resulting in improving the mechanical strength and the catalytic activity.

The amount of the binder used is generally 1 to 60 parts based on 100 parts of the preliminary calcination powder. The amount of the crystalline cellulose used for the binder is preferably 1 to 10 parts, more preferably 2 to 6 parts based on 100 parts of the preliminary calcination powder.

A shaping auxiliary such as silica gel, diatomaceous earth and alumina powder may be used if necessary in the present invention. The amount of shaping auxiliary used is generally 5 to 60 parts based on 100 parts of the preliminary calcination powder.

An inorganic fiber such as ceramic fiber and whisker may be used for a strength-improver if necessary, effecting to increase the mechanical strength of the catalyst. However, a fiber such as potassium titanate whisker and basic magnesium carbonate whisker is not preferable since it is reactive with the catalytic components. Ceramic fiber is particularly preferable. The amount of the fiber used is generally 1 to 30 parts based on 100 parts of the preliminary calcination powder.

The above shaping auxiliary and strength-improver are generally mixed with the preliminary calcination powder to use.

The coated product obtained by coating the preliminary calcination powder on a carrier has generally a diameter of 3 to 15mm.

The coated product thus obtained can be calcined primarily to get the coated catalyst being an object. The primary calcinations is generally carried out at a temperature of 250 to 500°C, preferably 300 to 450°C, for 1 to 50 hrs.

The size of the coated catalyst is different by the carrier which is used and a coating amount of the catalyst etc. It is generally about 3 to 15 mm and preferably nearly the same as the size of the carrier which is used or a little larger than that. The preferable size of the coated catalyst is 1.3 time of that of the carrier or less, preferably 1.2 time or less, further preferably 1.1 time or less.

EXAMPLES

The present invention will be described in more detail by way of the following examples and comparative examples. However, the present invention shall not be limited to these examples unless they are beyond the gist of the present invention.

Acrolein conversion, acrylic acid selectivity, and acrylic acid yield are defined by the following equations (2) to (4) respectively.

$$(2) \ \text{Acrolein conversion (\% by mol)=}$$

$$100 \times (\text{acrolein reacted in mole})/(\text{acrolein supplied in mole})$$

$$(3) \ \text{Acrylic acid selectivity (\% by mol)=}$$

$$100 \times (\text{acrylic acid produced in mole})/(\text{acrolein converted in mole})$$

```
(4) Acrylic acid yield (% by mol)=

    100×(acrylic acid produced in mole)/(acrolein supplied in

    mole)
```

Attrition resistance was measured with the tablet attrition resistance tester made by KAYAGAKI IRIKA KOGYO KK. A Catalyst sample put in the tester was rotated at 25rpm for 10min and screened through a 2 .36mm standard sieve. The mass of catalyst sample remaining on the sieve was determined to calculate the attrition resistance by the equation (5):

```
(5) Attrition resistance (% by mass)=

    100×(mass of sample put - mass of sample remaining on 2.36mm

    sieve)/( mass of sample put)
```

Antimony acetate having a purity of 99.6% was used in the examples.

Example 1 (reference)

In a formulating tank (A) equipped with a stirring motor, 600 parts of 95°C deionized water and 16.26 parts of ammonium tungstate were put and stirred. 18.22 parts of ammonium metavanadate and 110 parts of ammonium molybdate were then added to dissolve. 7.75 parts of antimony acetate (purity 99.6%)was addedfurther. In 96 parts of deionized water held in a formulating tank (B), 15.56 parts of copper sulfate were dissolved and the resultant solution was added into the formulating tank (A) to get a slurry solution.

The slurry solution was spray-dried under adjusting the feed rate so that the temperature at the outlet of the spray drier might be kept at about 100°C. The granule thus obtained was calcined (preliminary calcination) at 390°C for about 5hrs in a furnace, of which the temperature had been risen at a rate of about 60°C per hour from the room temperature. Then, the granule thus obtained (hereinafter in the examples called preliminary calcination granule) was pulverized in a ball mill to get a powder (hereinafter in the examples called preliminary calcination powder).

In a tumble granulator, 12 parts of the preliminary calcination powder was placed on 36 parts of alundum carrier having a porosity rate of 40%, a water-absorption rate of 19.8% and a diameter of 4mm while sprinkling 2.4 parts of 20% aqueous glycerin solution. The shaped product thus obtained was calcined at 390°C for 5hrs in a furnace, of which the temperature had been risen at a rate of about 70°C per hour from the room temperature, to get a coated catalyst The coated catalyst had almost the same particle size as the carrier. The atomic ratio of the catalytically active components excluding oxygen in the coated catalyst thus obtained is as follows:

$$Mo_{12}V_3W_{1.2}Cu_{1.2}Sb_{0.5}$$

The attrition resistance of this coated catalyst was 0.1% or less.

30ml of the coated catalyst thus obtained was packed into a reaction tube having an inner diameter of 21.4mm.

Propylene was applied to the vapor-phase catalytic oxidation using a molybdenum-bismuth catalyst in a reaction bath containing heat medium to get a gas, to which oxygen and nitrogen were supplemented to obtain a composite gas as described below.

| | |
|---|---|
| Acrolein | 5.5% by volume |
| Unreacted propylene and the other organic compounds | 1.3% by volume |
| Oxygen | 7.4% by volume |
| Steam | 27.0% by volume |
| Inert gas including nitrogen | 58.8% by volume |

The composite gas was made to pass through the above reaction tube at a SV (Space Velocity: Volume of flowing gas per unit time/Volume of packed catalyst) of 1800/hr to react.

The results of reaction at a reaction bath temperature of 245°C were as follows:

Acrolein conversion=99.2%
Acrylic acid selectivity=98.7%
Acrylic acid yield=97.9%

Comparative Example 1

A coated catalyst was obtained by the same way as described in Example 1, except that 3.78 parts of antimony trioxide were used in place of 7.75 parts of antimony acetate in Example 1. The atomic ratio of the catalytically active components excluding oxygen in the coated catalyst thus obtained is as follows:

$$Mo_{12}V_3W_{1.2}Cu_{1.2}Sb_{0.5}$$

The attrition resistance of this coated catalyst was 0.3%. The coated catalyst thus obtained was subjected to the reaction test in the same way as described in Example 1.
The results of reaction at a reaction bath temperature of 250°C were as follows:

Acrolein conversion=99.1%
Acrylic acid selectivity=98.5%
Acrylic acid yield=97.6%

Example 2 (reference)

23.1 parts of the preliminary calcination powder obtained in Example 1 were placed on 25 parts of alundum carrier having a porosity rate of 34%, a water-absorption rate of 17% and a diameter of 3.5mm while sprinkling 3.1 parts of 20% aqueous glycerin solution. The shaped product thus obtained was calcined at 390°C for 5hrs in a furnace the temperature of which had been programmed to rise at a rate of about 70°C per hour from the room temperature to get a coated catalyst The coated catalyst had an almost same particle size as the carrier. The atomic ratio of the catalytically active components excluding oxygen in the coated catalyst thus obtained is as follows:

$$Mo_{12}V_3W_{1.2}Cu_{1.2}Sb_{0.5}$$

The attrition resistance of this coated catalyst was 0.4%.

Comparative Example 2

A preliminary calcination powder was obtained by the same way as described in Example 1, except that 3.78 parts of antimony trioxide were used in place of 7.75 parts of antimony acetate in Example 1. 23.1 parts of the preliminary calcination powder thus obtained were placed on 25 parts of alundum carrier having a diameter of 3.5 mm while sprinkling 3.1 parts of 20% aqueous glycerin solution. The shaped product thus obtained was calcined at 390°C for 5hrs in a furnace the temperature of which had been programmed to rise at a rate of about 70°C per hour from the room temperature to get a coated catalyst. The diameter of the coated catalyst was 3.7 mm. The atomic ratio of the catalytically active components excluding oxygen in the coated catalyst thus obtained is as follows: $Mo_{12}V_3W_{1.2}Cu_{1.2}Sb_{0.5}$
The attrition resistance of this coated catalyst was 2.5%.

Example 3

15.4 parts of the preliminary calcination powder obtained in Example 1 were homogeneously mixed with 0.77 parts of crystalline cellulose. This mixed powder was placed on 36 parts of the same alundum carrier having a diameter of 3.5mm as used in Example 2 while sprinkling 2.6 parts of 20% aqueous glycerin solution. The shaped product thus obtained was calcined at 390°C for 5hrs in a furnace the temperature of which had been programmed to rise at a rate of about 70°C per hour from the room temperature to get a coated catalyst of the present invention. The diameter of the coated catalyst was 3.6 mm. The attrition resistance of this coated catalyst was 0.2%. The coated catalyst thus obtained was subjected to the reaction test in the same way as described in Example 1. The results of reaction at a reaction bath temperature of 235°C were as follows
Acrolein conversion=98.6%
Acrylic acid selectivity=98.9%

Acrylic acid yield=97.5%

**Example 4**

In a formulating tank (A) equipped with a stirring motor, 600 parts of 95°C deionized water and 16.26 parts of ammonium tungstate were put and stirred. 18.22 parts of ammonium metavanadate and 110 parts of ammonium molybdate were then added to dissolve. 7.75 parts of antimony acetate was added further. In 96 parts of deionized water held in a formulating tank (B), 23.33 parts of copper sulfate and 1.05 parts of potassium nitrate were dissolved and the resultant solution was added into the formulating tank (A) to get a slurry solution.

The slurry solution was spray-dried under adjusting the feed rate so that the temperature at the outlet of a spray drier might be kept at about 100°C to get a dried granule . The granule thus obtained was calcined at 390°C for about 5hrs in a furnace, of which the temperature had been risen at a rate of about 60°C per hour from the room temperature.

The preliminary calcination granule thus obtained was pulverized in a ball mill to get a preliminary calcination powder 12 parts of the preliminary calcination powder thus obtained were homogeneously mixed with 0.77 parts of crystalline cellulose.

The above mixed powder was placed on 36 parts of the same alundum carrier having a diameter of 4mm as used in Example 1 while sprinkling 2.6 parts of 20% aqueous glycerin solution. The shaped product thus obtained was calcined at 390°C for 5hrs in a furnace, of which the temperature had been risen at a rate of about 70°C per hour from the room temperature, to get a coated catalyst of the present invention. The atomic ratio of the catalytically active components excluding oxygen in the coated catalyst thus obtained is as follows:

$$Mo_{12}V_3W_{1.2}Cu_{1.8}Sb_{0.5}K_{0.2}$$

The diameter of the coated catalyst was almost the same as that of the carrier. The attrition resistance of this coated catalyst was 0.1% or less.

The coated catalyst was subjected to the reaction test in the same way as described in Example 1.

The results of reaction at a reaction bath temperature of 240°C were as follows

Acrolein conversion=98.6%

Acrylic acid selectivity=98.4%

Acrylic acid yield=97.0%

Example 5 (reference)

A coated catalyst of the present invention was obtained by the same way as described in Example 4, except that no cellulose was used.

The atomic ratio of the catalytically active components excluding oxygen in the coated catalyst thus obtained is as follows:

$$Mo_{12}V_3H_{1.2}Cu_{1.8}Sb_{0.5}K_{0.2}$$

The attrition resistance of this coated catalyst was 0.1% or less.

The coated catalyst was subjected to the reaction test in the same way as described in Example 1.

The results of reaction at a reaction bath temperature of 245°C were as follows:

Acrolein conversion=98.5%

Acrylic acid selectivity=98.1%

Acrylic acid yield=96.5%

Example 6

A coated catalyst of the present invention was obtained by the same way as described in Example 4, except that 3.99 parts of magnesium nitrate were used in place of 1.05 parts of potassium nitrate.

The atomic ratio of the catalytically active components excluding oxygen in the coated catalyst thus obtained is as follows:

$$Mo_{12}V_3W_{1.2}Cu_{1.8}Sb_{0.5}\,Mg_{0.3}$$

The attrition resistance of this coated catalyst was 0.1% or less.

The coated catalyst was subjected to the reaction test in the same way as described in Example 1.

The results of reaction at a reaction bath temperature of 245°C were as follows:

Acrolein conversion=98.7%
Acrylic acid selectivity=98.0%
Acrylic acid yield=96.7%

Example 7

In a formulating tank (A) equipped with a stirring motor, 600 parts of 95°C deionized water and 16.26 parts of ammonium tungstate were put and stirred. 18.22 parts of ammonium metavanadate and 110 parts of ammonium molybdate were then added to dissolve. 7.75 parts of antimony acetate was added further. After 20min, 2.07 parts of niobium oxide was added. In 96 parts of deionized water held in a formulating tank (B), 15.05 parts of copper nitrate was dissolved and the resultant solution was added into the formulating tank (A) to get a slurry solution.

The slurry solution was spray-dried under adjusting the feed rate so that the temperature at the outlet of the spray drier might be kept at about 100°C to get a dried granule. The granule thus obtained was calcined at 370°C for about 5hrs in a furnace, of which the temperature had been risen at a rate of about 60°C per hour from the room temperature.

The preliminary calcination granule thus obtained was pulverized in a ball mill to get a preliminary calcination powder. 12 parts of the preliminary calcination powder thus obtained was homogeneously mixed with 0.77 parts of crystalline cellulose.

The above mixed powder was placed on 36 parts of the same alundum carrier having a diameter of 4mm as used in Example 1 while sprinkling 2.7 parts of 6% aqueous glycerin solution. The shaped product thus obtained was calcined at 390°C for 5hrs in a furnace, of which the temperature had been risen at a rate of about 70°C per hour from the room temperature, to get a coated catalyst. The atomic ratio of the catalytically active components excluding oxygen in the coated catalyst thus obtained is as follows:

$$Mo_{12}V_3 W_{1.2} Cu_{1.2} Sb_{0.5}Nb_{0.3}$$

The attrition resistance of this coated catalyst was 0.1% or less.

The coated catalyst was subjected to the reaction test in the same way as described in Example 1.

The results of reaction at a reaction bath temperature of 243°C were as follows:

Acrolein conversion=99.0%
Acrylic acid selectivity=98.4%
Acrylic acid yield=97.4%

Example 8

Inaformulatingtank (A) equipped with a stirring motor, 600 parts of 95°C deionized water and 16.26 parts of ammonium tungstate were put and stirred. 18.22 parts of ammonium metavanadate and 110 parts of ammonium molybdate were then added to dissolve. 7.75 parts of antimony acetate was added further. In 96 parts of deionized water held in a formulating tank (B), 15.56 parts of copper sulfate, 0.52 parts of potassium nitrate and 4.19 parts of ferric nitrate were dissolved and the resultant solution was added into the formulating tank (A) to get a slurry solution.

The slurry solution was spray-dried under adjusting the feed rate so that the temperature at the outlet of a spray drier might be kept at about 100°C to get a dried granule. The granule thus obtained was calcined at 370°C for about 5hrs in a furnace, of which the temperature had been risen at a rate of about 60°C per hour from the room temperature.

The preliminary calcination granule thus obtained was pulverized in a ball mill to get a preliminary calcination powder. 12 parts of the preliminary calcination powder thus obtained was homogeneouslymixedwith 0.77 parts of crystalline cellulose.

The above mixed powder was placed on 36 parts of the same alundum carrier having a diameter of 4 mm as used in Example 1 while sprinkling 2.6 parts of 20% aqueous glycerin solution. The shaped product thus obtained was calcined at 390°C for 5hrs in a furnace, of which the temperature had been risen at a rate of about 70°C per hour from the room temperature, to get a coated catalyst of the present invention. The atomic ratio of the catalytically active components excluding oxygen in the coated catalyst thus obtained is as follows:

$$Mo_{12}V_3W_{1.2}Cu_{1.2}Sb_{0.5}K_{0.1}Fe_{0.2}$$

The attrition resistance of this coated catalyst was 0.1% or less.

The coated catalyst was subjected to the reaction test in the same way as described in Example 1.

The results of reaction at a reaction bath temperature of 243°C were as follows:

Acrolein conversion=98.7%
Acrylic acid selectivity=98.7%
Acrylic acid yield=97.4%

Example 9 (reference)

18 parts of the preliminary calcination powder obtained in Example 1 was homogeneously mixed with 0.9 parts of silica alumina fiber having an average fiber length of 100 $\mu$m as well as an average fiber diameter of 2.0 $\mu$m and 0.54 parts of methyl cellulose. In a tumble granulator, the above mixed powder was placed on 35.1 parts of the same alundum carrier having a diameter of 3.5mm as used in Example 2 while sprinkling 2.8 parts of 20% aqueous glycerin solution. The shaped product thus obtained was calcined at 390°C for 2.5hrs in a furnace, of which the temperature had been risen at a rate of about 70°C per hour from the room temperature, to get a coated catalyst.

The attrition resistance of this coated catalyst was 0.1% or less.

The coated catalyst was subjected to the reaction test in the same way as described in Example 1.

The results of reaction at a reaction bath temperature of 230°C were as follows:

Acrolein conversion=99.4%
Acrylic acid selectivity=98.4%
Acrylic acid yield=97.8%

INDUSTRIAL APPLICABILITY

The coated catalyst produced according to the present invention can be used in the process for producing an unsaturated acid from the unsaturated aldehyde as a source material, preferably in the process for producing acrylic acid from acrolein as the source material.

The catalyst produced according to the present invention is industrially valuable because it has a large mechanical strength enough to pack in a reaction tube with the catalytically active component little peeled and pulverized and because it has a high acrylic acid selectivity enough to cope with a high load reaction condition.

**Claims**

1. A process for producing a coated catalyst by coating the catalytically active component which has a composition represented by the formula (1):

$$Mo_{12}V_aW_bCu_cSb_dX_eY_fZ_gO_h \qquad (1)$$

wherein Mo, V , W , Cu, Sb and O represent molybdenum, vanadium, tungsten, copper, antimony and oxygen respectively;

X represents at least one element selected from the group consisting of alkali metals and thallium;

Y represents at least one element selected from the group consisting of magnesium, calcium, strontium, barium and zinc;

Z represents at least one element selected from the group consisting of niobium, cerium, tin, chromium, manganese, iron, cobalt, samarium, germanium, titanium and arsenic;

a, b, c, d, e, f, g and h represent atomic ratios of their respective elements, with $0 < a \leq 10, 0 \leq b \leq 10, 0 < c \leq 6, 0 < d \leq 10, 0 \leq e \leq 0.5, 0 \leq f \leq 1, 0 \leq g < 6$, based on 12 of molybdenum atoms; and h is the number of oxygen atoms required to satisfy the total valence of the other elements, on an inactive carrier,

the process comprising the following steps (a) to (c):

(a) drying either an aqueous solution or a dispersion containing the compounds of the catalytic component elements, wherein the aqueous solution or dispersion contains antimony acetate as the antimony material, thus obtaining a catalytic component composition;

(b) calcining the catalytic component composition obtained in step (a), to prepare a calcined powder; and

(c) coating the calcined powder obtained in step (b) on the carrier, together with crystalline cellulose as a binder and optionally a strength-improver.

2. The process according to claim 1, wherein the antimony acetate as the antimony source material for the preparation of the aqueous solution or the dispersion has a purity of 99 % or more.

3. The process according to claim 1 or 2, wherein the supported-calcined powder has 15 to 50% by mass rate based on the total amount of said carrier and said calcined powder in the step (c).

4. The process according to any of claims 1 to 3, wherein said drying in step (a) is spray-drying.

5. The process according to any of claims 1 to 4, wherein said strength-improver in the step (c) is ceramic fiber.

6. Use of the coated catalyst according to any of the previous claims in a process for the production of acrylic acid by vapor-phase catalytic oxidation of acrolein with molecular oxygen.

**Patentansprüche**

1. Ein Verfahren zur Herstellung eines beschichteten Katalysators durch Beschichten der katalytisch aktiven Komponente, die eine durch die Formel (1) dargestellte Zusammensetzung hat:

$$Mo_{12}V_aW_bCu_cSb_dX_eY_fZ_gO_h \qquad (1)$$

worin Mo, V, W, Cu, Sb und O Molybdän, Vanadium, Wolfram, Kupfer, Antimon und Sauerstoff jeweils darstellen; X mindestens ein Element darstellt, das ausgewählt ist aus der Gruppe, bestehend aus Alkalimetallen und Thallium; Y mindestens ein Element darstellt, das ausgewählt ist aus der Gruppe, bestehend aus Magnesium, Calcium, Strontium, Barium und Zink; Z mindestens ein Element darstellt, das ausgewählt ist aus der Gruppe, bestehend aus Niob, Cer, Zinn, Chrom, Mangan, Eisen, Kobalt, Samarium, Germanium, Titan und Arsen; a, b, c, d, e, f, g und h, die Atomverhältnisse ihrer jeweiligen Elemente darstellen, mit $0 < a \leq 10$, $0 \leq b \leq 10$, $0 < c \leq 6$, $0 < d \leq 10$, $0 \leq e \leq 0,5$, $0 \leq f \leq 1$, $0 \leq g < 6$, bezogen auf 12 Molybdänatome; und h die Anzahl der Sauerstoffatome ist, die benötigt werden, um die Gesamtwertigkeit der anderen Elemente auf dem inaktiven Träger zu erfüllen, wobei das Verfahren die folgenden Stufen (a) bis (c) umfasst:

(a) Trocknen entweder einer wässrigen Lösung oder Dispersion, enthaltend die Verbindungen der Elemente der katalytischen Komponente, worin die wässrige Lösung oder Dispersion Antimonacetat als Antimonmaterial enthält, um so die Zusammensetzung der katalytischen Komponente zu erhalten;
(b) Kalzinieren der in Stufe (a) erhaltenen Zusammensetzung der katalytischen Komponente, um ein kalziniertes Pulver herzustellen; und
(c) Beschichten des in Stufe (b) erhaltenen kalzinierten. Pulvers auf den Träger, zusammen mit kristalliner Zellulose als Bindemittel und gegebenenfalls einem Festigkeitsverbesserungsmittel,

2. Das Verfahren gemäß Anspruch 1, bei dem das Antimonacetat als Antimon-Ausgangsstoff zur Herstellung der wässrigen Lösung oder Dispersion eine Reinheit von 99% oder mehr hat.

3. Das Verfahren gemäß Anspruch 1 oder 2, bei dem das gestützte kalzinierte Pulver 15 bis 50% Masseverhältnis, bezogen auf die Gesamtmenge des Trägers und kalzinierten Pulvers in der Stufe (c), hat.

4. Das Verfahren gemäß einem der Ansprüche 1 bis 3, bei dem die Trocknung in Stufe (a) Sprühtrocknen ist.

5. Das Verfahren gemäß einem der Ansprüche 1 bis 4, bei dem das Festigkeitsverbesserungsmittel in der Stufe (c) keramische Faser ist.

6. Verwendung des beschichteten Katalysators gemäß einem der vorstehenden Ansprüche in einem Verfahren zur Herstellung von Acrylsäure durch katalytische Dampfphasenoxidation von Acrolein mit molekularem Sauerstoff.

**Revendications**

1. Procédé pour produire un catalyseur enrobé, en enrobant le composant catalytiquement actif qui a une composition représentée par la formule (1) :

$$Mo_{12}V_aW_bCu_cSb_dX_eY_fZ_gO_h \qquad (1)$$

dans laquelle Mo, V, W, Cu, Sb et O représentent respectivement le molybdène, le vanadium, le tungstène, le cuivre, l'antimoine et l'oxygène ;

X représente au moins un élément choisi dans le groupe constitué par les métaux alcalins et le thallium ;

Y représente au moins un élément choisi dans le groupe constitué par le magnésium, le calcium, le strontium, le baryum et le zinc ;

Z représente au moins un élément choisi dans le groupe constitué par le niobium, le cérium, l'étain, le chrome, le manganèse, le fer, le cobalt, le samarium, le germanium, le titane et l'arsenic ;

a, b, c, d, e, f, g et h représentent les rapports atomiques de leurs éléments respectifs, avec $0 < a \leq 10$, $0 \leq b \leq 10$, $0 < c \leq 6$, $0 < d \leq 10$, $0 \leq e \leq 0,5$, $0 \leq f \leq 1$, $0 \leq g < 6$, sur la base de 12 atomes de molybdène ; et h est le nombre d'atomes d'oxygène requis pour satisfaire à la valence totale des autres éléments, sur un support inactif, le procédé comprenant les étapes (a) à (c) suivantes :

(a) sécher une solution ou une dispersion aqueuse contenant les composés des éléments du composant catalytique, dans lequel la solution ou dispersion aqueuse contient de l'acétate d'antimoine comme matériau antimoine, obtenant de cette manière une composition de composant catalytique ;

(b) calciner la composition de composant catalytique obtenue à l'étape (a), pour préparer une poudre calcinée ; et

(c) revêtir le support de la poudre calcinée obtenue à l'étape (b), avec de la cellulose cristalline comme liant, et éventuellement un agent améliorant la résistance.

2. Procédé selon la revendication 1, dans lequel l'acétate d'antimoine comme matériau source d'antimoine pour la préparation de la solution ou dispersion aqueuse, a une pureté de 99 % ou plus.

3. Procédé selon la revendication 1 ou 2, dans lequel la poudre calcinée supportée a un rapport massique de 15 à 50 % basé sur la quantité totale dudit support et de ladite poudre calcinée à l'étape (c).

4. Procédé selon l'une quelconque des revendications 1 à 3, dans lequel ledit séchage de l'étape (a) est un séchage par pulvérisation.

5. Procédé selon l'une quelconque des revendications 1 à 4, dans lequel ledit agent améliorant la résistance de l'étape (c) est de la fibre céramique.

6. Utilisation du catalyseur enrobé selon l'une quelconque des revendications précédentes dans un procédé pour produire de l'acide acrylique par oxydation catalytique en phase vapeur d'acroléine avec de l'oxygène moléculaire.

**EP 1 138 385 B1**

**REFERENCES CITED IN THE DESCRIPTION**

**Patent documents cited in the description**

- FR 2754817 A
- EP 0711745 A
- JP 51011709 A
- JP 52153889 A
- JP 52153889 B
- JP 1085139 A
- JP 8299797 A
- JP 8299797 B
- EP 0758562 A